(19) Europäisches Patentamt

European Patent Office

Office européen des brevets

(11) **EP 1 591 074 B1**

(12) **EUROPEAN PATENT SPECIFICATION**

(45) Date of publication and mention
of the grant of the patent:
**21.05.2008 Bulletin 2008/21**

(51) Int Cl.:
***A61B 19/00*** *(2006.01)*

(21) Application number: **04009835.2**

(22) Date of filing: **26.04.2004**

(54) **Visualization of procedural guidelines for medical procedures**

Visualisierung von Verfahrensrichtlinien für medizinische Verfahren

Visualisation de directives procédurales pour procédures médicales

(84) Designated Contracting States:
**DE FR GB IT**

(43) Date of publication of application:
**02.11.2005 Bulletin 2005/44**

(73) Proprietor: **BrainLAB AG**
**85551 Kirchheim/Heimstetten (DE)**

(72) Inventors:
• **Birkenbach, Rainer**
**85445 Aufkirchen (DE)**
• **Pedain, Christoph**
**81667 Munich (DE)**
• **Hartlep, Andreas, Dr.**
**83629 Naring (DE)**
• **Raghavan, Raghu**
**Baltimore, MD 21210 (US)**
• **Brady, Martin**
**Phoenix, MD 21131 (US)**

(74) Representative: **Schwabe - Sandmair - Marx**
**Stuntzstrasse 16**
**81677 München (DE)**

(56) References cited:
**EP-A- 1 103 229    EP-A- 1 199 031**
**EP-A- 1 410 758    US-B1- 6 381 483**

**Description**

The present invention relates to a method for visualizing procedural guidelines for positioning a catheter for administrating substances to a body portion. In particular, the present invention deals with procedures In which a catheter is shown in an image representation of a body portion including an area in which the administration is to be carried out.

For many medical procedures, certain rules or guidelines exist or may be established. One example would be a guideline or rule determining where the tip of an instrument should be placed in order to achieve an optimum effect. Another guideline may determine locations where such an instrument is not to be placed, in order to prevent harm to the patient or undesired effects.

Medical technology has developed to a point where surgeons may use so-called image-guided surgery systems, displaying image representations of body portions as well as instruments in their correct positional relationship to said image representations. At present, surgeons must try to keep procedural guidelines in mind, while they operate in an image-guided environment.

A problem arising from this practice resides in the fact that a surgeon may only be able to remember or take into account a few guidelines at a time, while he is carrying out a complicated operation. Also, the constraints given by such guidelines may be very precise, whereas a surgeon equipped with the information of the image representation may only be able to guess whether such constraints are being fulfilled at a certain moment.

US 6,381,483 B1 discloses a therapeutic system comprising a therapeutic applicator for treating living tissue and an observation unit for determining the position of the applicator. In said system the therapeutic energy applied from the applicator to the living tissue is controlled in accordance with data representing the position of the applicator, determined by the observation unit.

EP 1 199 031 A2 discloses the determination of the position of a medical instrument in an object to be examined. The medical instrument has a localizing device on its distal end, which serves to determine the position of the instrument. It further comprises an image detection device for gathering information in the vicinity of the instrument which image information is displayed together with the instrument position.

From EP 1 410 758 A1 an endoscopic image pick-up method is known, wherein a catheter is inserted into a body cavity, a three-dimensional MR image is taken and the position and direction of the extreme end of the catheter on the MR image are detected, the center project image is reconstructed and the tip position is set.

EP 1 103 229 A2 and US 5,638,819 disclose systems and methods for planning interventional procedures, wherein planned or desired trajectories to a target are displayed on medical images of a patient.

It is an object of the present invention to provide an aid for ensuring that certain guidelines for a substance administration can easily be followed. In particular, it is an object of the invention to provide easily interpretable visual information relating to such guidelines.

This object is achieved by a method according to claim 1. The sub-claims define advantageous embodiments of the present invention.

The advantages of the present invention arise from the fact that, in the vicinity of the functional portion of the catheter, regions or locations are indicated which are of special relevance to the administration procedure according to established guidelines for said procedure. Said regions or locations may be displayed in or overlaid on the image representation of the body portion and the catheter, thus giving the user direct visual information as to whether his presently planned course of action, e.g. the presently planned trajectory of the catheter, would fulfill the constraints given by the guidelines, or not. The surgeon does not have to occupy himself with remembering all the necessary rules or guidelines and he does not have to do any guess-work as to whether certain rules or guidelines are being accurately obeyed. The surgeon can concentrate on other aspects of his work and, in particular, can rely on the visual information if he is not able to determine whether the constraints given by the rules or guidelines are being fulfilled, by experience alone. The guidelines determine regions in which:

- the delivered substance is expected to spread;
- the delivering portion of the catheter is to be placed, in order to exhibit a sufficient distance from critical tissue portions;
- the delivering portion of the catheter is to be placed, in order to exhibit a sufficient distance from other delivering portions of other catheters;

wherein one or more of the above regions may be displayed.

In a preferred embodiment of the present invention, said catheter and said body portion are positionally tracked by a medical tracking system, acquired anatomical body data being correlated with the tracking information by an image-guided surgery system, and said image representation being a screen output of said image-guided surgery system.

There are, in principle, two ways of determining said regions or locations of special relevance. One way is to develop and fixedly set of standard guidelines for a certain medical procedure. In this case, said standard guidelines would consist of predetermined values which may have proven to offer good effects in certain comparable procedures.

Another possibility is to determine said regions or locations of special relevance by applying guidelines computed for a current procedure on the basis of instrument and body data. This way, achieving a basis for the visualization may require a greater effort in the beginning, but will provide better case-adapted information.

Said regions or locations of special relevance may, according to the present invention, be indicated as regions or locations in which, according to the guidelines:

- an administration is to be applied;
- an administration is not to be applied;
- an administration is preferably to be applied;
- an administration is preferably not to be applied;
- an administration will show an effect;
- an administration will not show an effect;

wherein one or more of the above indications can be displayed.

In one embodiment of the present invention, the medical procedure may be the positioning of a catheter for administrating substances to a body portion, in particular to a brain portion, wherein the functional portion of the instrument is the portion of the catheter by which the substance is delivered. In such a case,

When fixed standard guidelines are used, such guidelines could preferably determine that:

- the delivered substance is expected to spread in a 2 to 3 cm, in particular 2.5 cm, long substantially cylindrical area backwards from the catheter tip;
- the delivering portion of the catheter is to be placed at a distance of 5 mm to 15 mm, in particular 10 mm, from critical tissue portions, in particular such tissue portions as hinder the spread of the substance or guide the substance to undesirable locations;
- the delivering portion of the catheter is to be placed at a distance of at least 2 cm, in particular 3 cm, from other delivering portions of other catheters.

If variable guidelines are used, such guidelines may be computed on the basis of one or more of the following instrument and/or body data:

- substance flow rate;
- catheter geometry;
- tissue elasticity;
- hydraulic conductivity of the tissue;

or on the basis of parameters derived from such data or measured directly, such as pressure gradient, velocity gradient or concentration gradient.

The invention will now be explained in greater detail by referring to a preferred embodiment and to the attached drawings. In the drawings:

Figure 1    is a representation of an instrument, namely a catheter for administrating substances into the brain, with a visualization of certain regions which are of relevance when a substance is administered;

Figure 2    is a representation showing how the instrument and the visualizations would appear overlaid on an image representation of a brain area; and

Figure 3    is a screenshot showing three representations as in Figure 2 but from three different viewpoints, and one perspective representation.

The present invention will now be described within the context of a particular embodiment, namely within the context of treating brain tumors by administrating a therapeutic substance via a catheter.

For the treatment of recurrent glioblastomas in the human brain, direct pharmaceutical therapies are currently under development. A prerequisite of sufficient local therapy is the capability of the therapeutic substance to reach the assigned target. Catheters are used to deliver the therapeutic substance to the target volume. The placement of the catheters is planned pre-operatively using a computerized planning system and previously acquired patient images and other patient data. To avoid failure in substance delivery, certain rules or guidelines for placing the catheter have to be obeyed. These rules or guidelines are defined by the supplier of the therapeutic substance and/or by experts in the field of catheter administration.

In accordance with the present invention, the rules/guidelines are transformed into visualizations of certain regions or locations in order to enable them to be displayed. Such visualizations are "attached" to a planned, displayed trajectory of the catheter and overlayed on anatomical and/or functional patient data. Visualizing and displaying these rules, e.g. minimal depth of catheter tips or minimal distance to intra-cranial surfaces like cavities and/or sulci, enables the planning surgeon to avoid mistakes when placing the catheter.

Various physical analyses have allowed the inventors of the present invention to theoretically describe the extent of backflow of an infused fluid along a catheter track.

A description of the mathematical models that were used follows. The physical parameters used in the model are listed below:

K    Tissue Hydraulic Conductivity
G    Tissue Shear Modulus
$\phi$    Extracellular Volume Fraction
$\lambda$    Tissue Effective Radius
$\mu$    Fluid Viscosity
Q    Flow Rate
r    Catheter Radius

In the simplest model, the tissue is assumed to be homogeneous with respect to hydraulic resistance and shear modulus, and the catheter is assumed to approximate an infinite cylinder. In this case, the backflow distance, L, is estimated using the following formula:

$$L = \frac{5}{3}\left(\frac{\pi}{120\mu}\right)^{1/5}\left(\frac{\ln(\lambda/r)}{2\pi\phi K}\right)^{4/5}\frac{Q^{3/5}r^{4/5}}{G^{3/5}}$$

More detailed models which better approximate the catheter geometry and account for variations in the tissue properties along the length of the catheter have also been developed.

The above models provide the opportunity of adjusting the display of backflow distances according to the infusion parameters and the catheter type chosen. Computing a pressure field furthermore makes it possible to accommodate the displayed rules for nearby pressure sinks. Based on an analysis of an MRI-diffusion tensor scan, the mathematical models make it possible to take into account the varying hydraulic conductivity of tissue surrounding the catheter and to adjust the guidelines thus displayed.

Moreover, based on experiments and/or theoretical analyses, rules of thumb for the backflow distance can be derived. As an example, such rules of thumb may be summarized as in the table below:

Table 1. Backflow distance (mm) for various flow rates and catheter radii.

|  | r=0.5mm | r=0.75mm | r = 1.0 mm | r=1.25mm |
|---|---|---|---|---|
| Q = 1.0 ($\mu$l/minute) | 6.0 | 7.7 | 9.1 | 10.4 |
| Q = 3.0 ($\mu$l/minute) | 11.7 | 14.9 | 17.7 | 20.1 |
| Q = 5.0 ($\mu$l/minute) | 15.8 | 20.3 | 24.0 | 27.3 |
| Q = 7.0 ($\mu$l/minute) | 19.4 | 24.8 | 29.4 | 33.4 |
| Q = 9.0 ($\mu$l/minute) | 22.5 | 28.8 | 34.2 | 38.9 |

(Using parameters K = $4 \times 10^{-8}$ cm$^4$/(dyne-sec), G = $1 \times 10^5$ dyne/cm$^2$, $\lambda$ = $1 \times 10^5$ cm, $\mu$ = $7.06 \times 10^{-3}$ g/(cm-sec).)

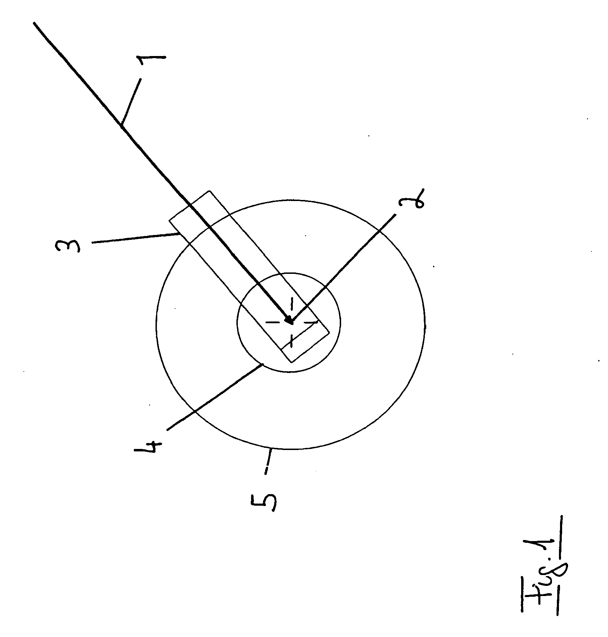

In one example, the backflow could be assumed to substantially fill a cylinder extending substantially backwards from the catheter tip and having a length of 2.5 cm. Given this premise, one guideline for catheter administration would be that this backflow area should substantially lie within the "white matter" tissue of the brain, preferably without contacting intra-cranial surfaces like cavities or the outer limits of the brain tissue. In Figure 1, the catheter has been given the reference numeral 1, and its tip is indicated by a small arrow and bears the reference numeral 2. As a visualization of the above guideline, a cylinder 3 can be displayed which extends from slightly ahead of the tip 2 backwards along the catheter 1, said cylinder being an elongated rectangle in the plan-view projection of Figure 1. Thus, the cylinder 3 visualizes the region where the substance is expected to spread after it has been administered and it therefore visualizes the guideline or transforms the guideline into a visualization, wherein said guideline rules that this cylinder should be in the white matter of the brain.

Another guideline is, for example, more directly visualized by two circles 4 and 5 centered on the tip 2. The first, inner circle 4 defines a region around the tip in which no cavity border of the brain tissue should be, whereas the circle 5 defines an area into which no tip of another catheter should be placed.

The circles in Figure 1 are, of course, representations of spheres having corresponding diameters. The inner sphere 4 may, for example, have a radius of 1 cm; the outer sphere 5 may have a radius of, for example, 3 cm. The length of the cylinder 1 may vary but in the present example should be in the range of about 4 cm.

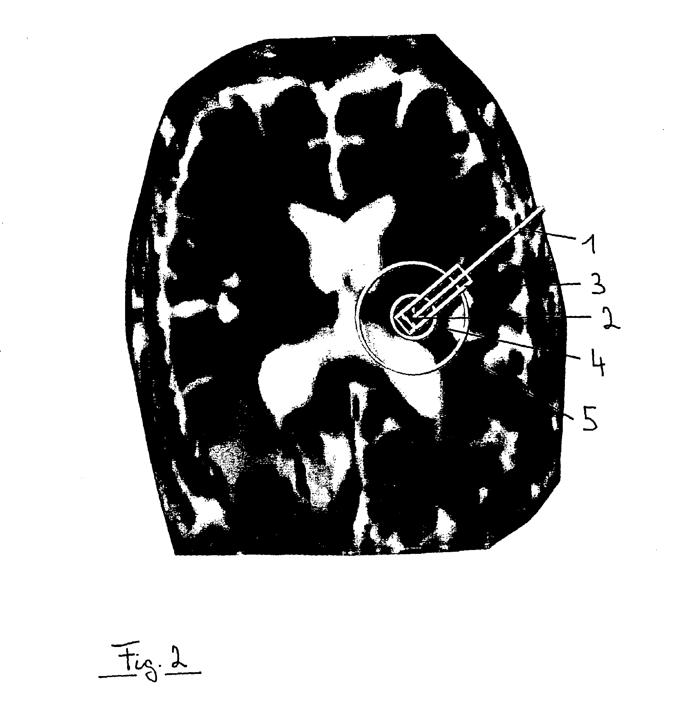

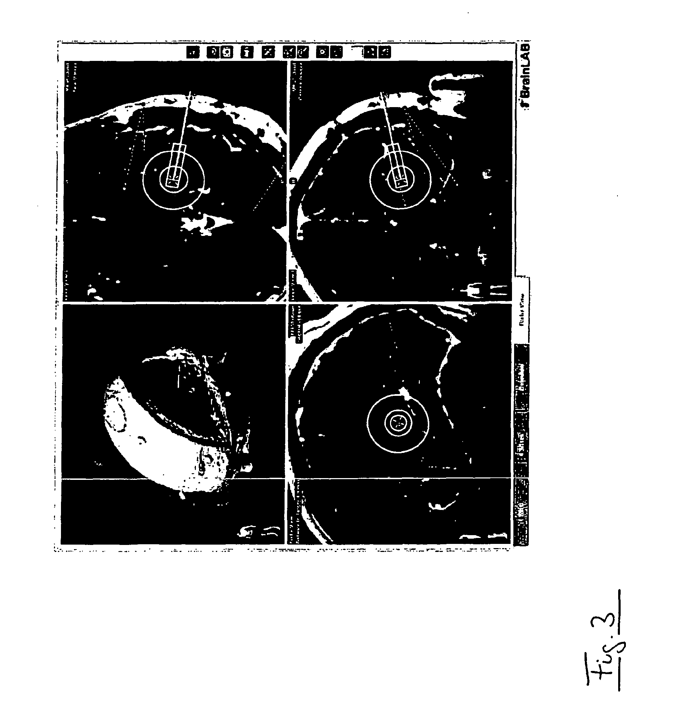

Figure 2 shows such a visualization of the rules/guidelines, i.e. a visualization of certain regions which are relevant according to the guidelines, in an MRI slice image of the skull/brain of a patient. It becomes obvious, that the catheter 1, together with its tip 2, can be visibly overlaid onto the anatomical image with the regions 3, 4 and 5 in such a way that it becomes very easy for a surgeon to decide whether this planned catheter positioning complies with or violates the guidelines. Of course, image representations like the one in Figure 2 can be displayed, from various angles alone or together, as well as in three dimensional perspective representations, to make sure that all of the spaces filled by the cylinder or the spheres are correctly positioned. The screenshot of Figure 3 shows such multiple representations. As soon as an acceptable position for the catheter 2, i.e. one obeying all or substantially obeying all the guidelines, has been found, this position can be determined as a suitable catheter placement plan.

In this respect, the invention also relates to a planning system that is able to display anatomical and/or functional and/or physiological patient-related image data. The system can be used to plan trajectories for the placement of intra-cranial ventricle catheters, for delivering substances into the human brain. If a trajectory is planned, then a certain catheter type and, if decided, a flow rate of the infusion can be assigned to the trajectory. Since the system knows the catheter type (e.g. diameter) and the flow rate, certain rules/guidelines for catheter placement can be displayed. For example, the minimal depth of the catheter tip can be displayed by a cylinder around the trajectory. The trajectory can be displayed in the thickness of the assigned catheter type. Minimal distances between intra-cranial surfaces and the catheter tip can be displayed by circles around the end of the trajectory. Distances between different catheter tips can be displayed by circles around the catheter tip having the diameter of the required distance. These visualizations can be matched onto anatomical and/or functional and/or physiological images of the patient.

All rules can be switched on and off. Additional rules can be applied. By using different colors or shades for certain visualizations, their degree of importance can be made visible. For example, very important rules/guidelines could be visualized in signal colors (red/yellow), whereas less important guidelines may be visualized in other colors. Moreover, the data included in the patient images could be used to determine whether a particular guideline is being fulfilled or not. For example, if it is determined by the image-guided surgery system that the sphere 4 (Figure 1 and 2) cuts through a region identified as a cavity in the brain, this sphere could then be displayed as flashing. Then, as soon as the planned trajectory has been moved to an adequate position, the flashing could stop, indicating that the sphere 4 is no longer contacting any brain cavity.

**Claims**

1. A method for visualizing procedural guidelines for positioning a catheter for administrating substances to a body portion, in which a catheter is shown in an image representation of the body portion including an area in which the administration is to be carried out, wherein, in the vicinity of the delivering portion of the catheter, regions or locations are indicated which are of special relevance to the administration according to established guidelines for said administration, wherein the guidelines determine regions in which:

   - the delivered substance is expected to spread;
   - the delivering portion of the catheter is to be placed, in order to exhibit a sufficient distance from critical tissue portions;
   - the delivering portion of the catheter is to be placed, in order to exhibit a sufficient distance from other delivering portions of other catheters;

   wherein one or more of the above regions may be displayed..

2. The method of claim 1, wherein said catheter and said body portion are positionally tracked by a medical tracking system, acquired anatomical body data being correlated with the tracking information by an image-guided surgery system, and said image representation being a screen output of said image-guided surgery system.

3. The method of claim 1 or 2, wherein said regions or locations of special relevance are determined by the application of fixedly set standard guidelines.

4. The method of claim 1 or 2, wherein said regions or locations of special relevance are determined by applying guidelines computed for a current procedure on the basis of instrument and body data.

5. The method of any one of claims 1 to 4, wherein said regions or locations of special relevance are indicated as regions or locations in which, according to the guidelines:

- an administration is to be applied;
- an administration is not to be applied;
- an administration is preferably to by applied;
- an administration is preferably not to be applied;
- an administration will show an effect;
- an administration will not show an effect;

wherein one or more of the above indications can be displayed.

6. The method of any one of claims 1 to 5, wherein said body portion is a brain portion.

7. The method of any one of claims 1 or 6, wherein the guidelines are fixed standard guidelines, determining that:

- the delivered substance is expected to spread in a 2 to 3 cm, in particular 2.5 cm, long, substantially cylindrical area backwards from the catheter tip;
- the delivering portion of the catheter is to be placed at a distance of 5 mm to 15 mm, in particular 10 mm, from critical tissue portions, in particular such tissue portions as hinder the spread of the substance or guide the substance to undesirable locations;
- the delivering portion of the catheter is to be placed at a distance of at least 2 cm, in particular 3 cm, from other delivering portions of other catheters.

8. The method of any one of claims 1 or 6, wherein the guidelines are variable guidelines computed on the basis of one or more of the following instrument and/or body data:

- substance flow rate;
- catheter geometry;
- tissue elasticity;
- hydraulic conductivity of the tissue;

or on the basis of parameters derived from such data or measured directly, such as pressure gradient, velocity gradient or concentration gradient.

**Patentansprüche**

1. Verfahren zum Visualisieren von Prozedur-Richtlinien zum Positionieren eines Katheters zum Verabreichen von Substanzen an ein Körperteil, bei welchem ein Katheter in einer Bilddarstellung des Körperteils mit einem Bereich, in dem die Verabreichung durchgeführt werden soll, gezeigt wird, wobei in der Umgebung des abgebenden Teils des Katheters Regionen oder Positionen, welche von besonderer Bedeutung für die Verabreichung gemäß bekannter Richtlinien der Verabreichung sind, angezeigt werden, wobei die Richtlinien Regionen bestimmen, in welchen:

- sich die abgegebene Substanz erwartungsgemäß ausbreitet;
- der abgebende Teil des Katheters platziert werden soll, um einen ausreichenden Abstand von kritischen Gewebeteilen aufzuweisen;
- der abgebende Teil des Katheters platziert werden soll, um einen ausreichenden Abstand von anderen abgebenden Teilen anderer Katheter aufzuweisen;

wobei eine oder mehrere der obigen Regionen dargestellt werden können.

2. Verfahren gemäß Anspruch 1, wobei der Katheter und die Körperregion positionell durch ein medizintechnisches Trackingsystem getrackt werden, ermittelte anatomische Körperdaten mit den Trackingdaten durch ein bildgeführtes Operationssystem korreliert werden und die Bilddarstellung eine Bildschirmausgabe des bildgeführten Operationssystems ist.

**3.** Verfahren gemäß Anspruch 1 oder 2, wobei die Regionen oder Positionen besonderer Bedeutung durch Anwendung festgelegter Standardrichtlinien bestimmt werden.

**4.** Verfahren gemäß Anspruch 1 oder 2, wobei die Regionen oder Positionen besonderer Bedeutung durch Anwenden von für ein durchgeführtes Verfahren auf der Basis von Instrument- und Körperdaten errechneten Richtlinien bestimmt werden.

**5.** Verfahren gemäß einem der Ansprüche 1 bis 4, wobei die Regionen oder Positionen besonderer Bedeutung als Regionen oder Positionen angezeigt werden, in welchen gemäß den Richtlinien:

- eine Verabreichung durchgeführt werden soll;
- eine Verabreichung nicht durchgeführt werden soll;
- eine Verabreichung vorzugsweise durchgeführt werden soll;
- eine Verabreichung vorzugsweise nicht durchgeführt werden soll;
- eine Verabreichung eine Auswirkung zeigen wird;
- eine Verabreichung keine Auswirkung zeigen wird;

wobei eine oder mehrere der obigen Anzeigen dargestellt werden können.

**6.** Verfahren gemäß einem der Ansprüche 1 bis 5, wobei der Körperteil Teil eines Gehirns ist.

**7.** Verfahren gemäß einem der Ansprüche 1 oder 6, wobei die Richtlinien festgelegte Standardrichtlinien sind, die ermitteln, dass:

- sich die abgegebene Substanz vermutlich in einem 2 bis 3 cm, im speziellen 2,5 cm langen, im Wesentlichen zylindrischen Bereich von der Katheterspitze aus nach hinten ausbreiten wird;
- der abgebende Teil des Katheters mit einem Abstand von 5 mm bis 15 mm, im speziellen 10 mm, von kritischen Gewebeteilen platziert werden soll, im speziellen von solchen Gewebeteilen, die der Ausbreitung der Substanz hinderlich sind oder die Substanz zu unerwünschten Positionen leiten;
- der abgebende Teil des Katheters mit einem Abstand von mindestens 2 cm, im Speziellen 3 cm, von anderen abgebenden Teilen anderer Katheter platziert werden soll.

**8.** Verfahren gemäß einem der Ansprüche 1 oder 6, wobei die Richtlinien variable Richtlinien sind, die auf der Basis einer oder mehrerer der folgenden Instrument- und/oder Körperdaten errechnet werden:

- Durchflussrate der Substanz;
- Kathetergeometrie;
- Gewebeelastizität;
- hydraulische Leitfähigkeit des Gewebes;

oder auf der Basis von Paramtern, die von solchen Daten abgeleitet oder direkt gemessenen werden, wie Druckgradient, Geschwindigkeitsgradient oder Konzentrationsgradient.

**Revendications**

**1.** Procédé pour visualiser des lignes directrices d'opération pour positionner un cathéter pour administrer des substances à une partie du corps, dans lequel un cathéter est montré dans une représentation d'image de la partie du corps comprenant une zone dans laquelle l'administration doit être effectuée, dans lequel, au voisinage de la partie de délivrance du cathéter, des régions ou des emplacements sont indiqués, ceux-ci ayant une pertinence particulière vis-à-vis de l'administration selon des lignes directrices établies pour ladite administration, dans lequel les lignes directrices déterminent des régions dans lesquelles :

- il est prévu que la substance délivrée diffuse ;
- la partie de délivrance du cathéter doit être disposée, afin de présenter une distance suffisante vis-à-vis de parties de tissu critiques ;

- la partie de délivrance du cathéter doit être disposée, afin de présenter une distance suffisante vis-à-vis d'autres parties de délivrance d'autres cathéters ;

dans lequel une ou plusieurs des régions ci-dessus peuvent être visualisées.

**2.** Procédé selon la revendication 1, dans lequel ledit cathéter et ladite partie du corps sont suivis en position par un système de suivi médical, des données corporelles anatomiques acquises étant corrélées avec l'information de suivi par un système de chirurgie guidée par une image, et ladite représentation d'image étant une sortie d'écran dudit système de chirurgie guidée par une image.

**3.** Procédé selon la revendication 1 ou 2, dans lequel lesdits emplacements ou régions de pertinence particulière sont déterminés par l'application de lignes directrices standard établies de façon fixe.

**4.** Procédé selon la revendication 1 ou 2, dans lequel lesdits emplacements ou régions de pertinence particulière sont déterminés par l'application de lignes directrices calculées pour une opération en cours en fonction d'un instrument et de données corporelles.

**5.** Procédé selon l'une quelconque des revendications 1 à 4, dans lequel lesdits emplacements ou région de pertinence particulière sont indiqués comme des emplacements ou régions dans lesquels, selon les lignes directrices :

- une administration doit être appliquée ;
- une administration ne doit pas être appliquée ;
- il est préférable qu'une administration soit appliquée ;
- il est préférable qu'une administration ne soit pas appliquée ;
- une administration produira un effet;
- une administration ne produira pas d'effet ;

dans lequel une ou plusieurs des indications ci-dessus peuvent être affichées.

**6.** Procédé selon l'une quelconque des revendications 1 à 5, dans lequel ladite partie du corps est une partie du cerveau.

**7.** Procédé selon l'une quelconque des revendications 1 ou 6, dans lequel les lignes directrices sont des lignes directrices standard fixes, déterminant que :

- il est prévu que la substance délivrée diffuse dans une zone sensiblement cylindrique de 2 à 3 cm, et, en particulier, de 2,5 cm, de long vers l'arrière à partir de la pointe du cathéter ;
- la partie de délivrance du cathéter doit être disposée à une distance de 5 mm à 15 mm, et, en particulier, de 10 mm, de parties de tissu critiques, en particulier des parties de tissu qui gênent la diffusion de la substance ou qui guident la substance vers des emplacements indésirables ;
- la partie de délivrance du cathéter doit être disposée à une distance d'au moins 2 cm, et, en particulier, de 3 cm, d'autres parties de délivrance d'autres cathéters.

**8.** Procédé selon l'une quelconque des revendications 1 à 5, dans lequel les lignes directrices sont des lignes directrices variables calculées en fonction d'un ou plusieurs parmi l'instrument et/ou les données corporelles suivants :

- le débit d'écoulement de la substance ;
- la géométrie du cathéter ;
- l'élasticité du tissu ;
- la conductivité hydraulique du tissu ;

ou en fonction de paramètres dérivés de ces données ou mesurés directement, tels qu'un gradient de pression, un gradient de vitesse ou un gradient de concentration.

Fig. 1

Fig. 2

EP 1 591 074 B1

Fig. 3

11

**EP 1 591 074 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6381483 B1
- EP 1199031 A2
- EP 1410758 A1
- EP 1103229 A2
- US 5638819 A